# EUROPEAN PATENT SPECIFICATION

(11) **EP 4 554 659 B1**
(45) Date of publication and mention of the grant of the patent: **30.09.2026**
(21) Application number: 23735335.4
(22) Date of filing: 29.06.2023
(51) Int. Cl.: A61N 1/36

(54) **NEUROLOGICAL STIMULATION SYSTEM**
NEUROLOGISCHES STIMULATIONSSYSTEM
SYSTÈME DE STIMULATION NEUROLOGIQUE

(30) Priority: 13.07.2022 US 202263388751 P; 09.08.2022 EP 22189541
(43) Date of publication of application: 21.05.2025
(73) Proprietor: BIOTRONIK SE & Co. KG, 12359 Berlin (DE)
(72) Inventor: PAMELA SHAMSIE VICTORIA, Riahi, Portland, Oregon 97232 (US); LIM, Koeun, Tucson, Arizona 85750 (US); KIBLER, Andrew B., Lake Oswego, Oregon 97035 (US)
(74) Representative: Biotronik Corporate Services SE
(86) International application number: PCT/EP2023/067809
(87) International publication number: WO 2024/012885

(56) References cited:
- US-A1- 2021 228 872
- US-A1- 2022 111 212
- US-A1- 2022 143 410

## Description

The present invention provides a neurological stimulation system according to claim 1 and machine-readable storage medium according to claim 11. Embodiments of the invention are defined in the dependent claims.

The present disclosure provides also further, non-claimed, examples of a method which can be carried out using the claimed system.

Spinal Cord Stimulation (SCS) is a treatment for chronic pain that uses electrical current sent through electrodes on one or multiple leads implanted in the spinal cord. Stimulation parameters may include a pulse width, frequency and/or amplitude. Additionally, duty cycling, program cycling and burst stimulation can be introduced to further modulate therapy and battery consumption by introducing alternating periods of carrier and envelope stimulation on and off.

Generally, all those parameters are set at least at initial therapy programming. The pool of possible parameter combinations is extensive given the number of parameters, and the choice of parameters vary with a range of factors such as manufacturers recommendations, therapy type (sub-perception or traditional), pain etiology/area location, lead location, patient anatomy, stimulation perception threshold, previous experience with e.g. a trial stimulator, etc.

Such high dimensional parameter space and nonlinear nature of the neural physiology pose two major difficulties in finding the optimal set of parameters. First, multiple local optima may exist, making the search for the global optimum difficult. Secondly, nonlinearity including discontinuity in the parameter space may cause the local tuning to diverge, driving the solution farther away from the optimum. Hence, the analytical solution of the optimal set of parameters is usually unknown, and parameter optimization is typically achieved empirically, for each patient, over time. This usually happens during a trial period, in which the patient is prescribed permanent implant only if they experience significant pain relief, usually 50% or more. This sparse, unguided sampling leads to potentially missing the effective parameter combination for a given patient. Additionally, a satisfying SCS therapy experience, e.g. a reduction of pain of 60%, qualifies as effective, but may not be the best SCS can achieve for this given patient, and the absolute optimal may not be reached with conventional manual optimization procedures. Furthermore, it should be taken into consideration that pain relief is the primary therapy target of SCS, but for achieving optimal therapy outcomes, other factors associated with quality of life or patient burden may also play a significant role, such as increase or decrease of a recharging time.

US 2021/228872 A1 describes a system for stimulation of a subject. The stimulation may be to provide therapy to treat the subject. Stimulation may be of selected muscle groups and/or portions.

Furthermore, US 2022/111212 A1 discusses brain monitoring and stimulation devices using parameter sets that are automatically refined during operation. For example, a system may comprise a plurality of stimulation devices connected to signal output circuitry interfacing the processor with the stimulation devices, so that the processor generates and transmits stimulation signals to the stimulation devices, and a plurality of sensing devices connected to signal input circuitry interfacing the processor with the sensing devices.

US 2022/0143410 A1 teaches a computing system for optimizing a waveform, in communication with an implantable pulse generator, and including a computing device including a memory device and a processor communicatively coupled to the memory device. The processor is configured to: retrieve historical waveform data associated with a plurality of waveforms used in therapeutic sessions for a plurality of patients, the historical waveform data including a plurality of waveform parameters; analyzing the historical waveform data to determine preferred waveform parameters; determining that a patient is starting a new therapeutic session using the patient therapeutic device; displaying each of the preferred waveform parameters; prompting the user to accept or modify the displayed waveform parameters; optimizing the waveform parameters for the therapeutic session; and transmitting the optimized waveform parameters to the patient therapeutic device to start the therapeutic session.

In light of the above, there is a need to reliably find an optimal set of parameters that achieve satisfying pain relief for each patient, and find the most optimal set of parameters that achieves the best pain relief a patient can experience with SCS, and preferably a set of parameters that also minimizes power consumption to promote the longevity of the stimulator battery as well as improve patient's overall experience by reducing charging frequency in case a rechargeable stimulator is used.

It is an object of the present disclosure to provide a method for neurological stimulation, a machine-readable storage medium for executing the method, and a neurological stimulation system, which can provide an efficient control of neurological stimulation therapy. A further object of the present disclosure is to improve the pain relief and/or therapy satisfaction of patients. A yet further object of the present disclosure is to minimize power consumption to promote the longevity of a battery as well as improving the patient's overall experience by reducing charging frequency in case a rechargeable stimulator is used.

According to an independent aspect of the present disclosure, a method for neurological stimulation is provided. The method includes determining (or optimizing), by a therapy parameter determination module, a plurality of therapy parameters using an algorithm which includes a generalized population model based on first patient data of multiple patients and an individualized model based on second patient data of the patient who uses the signal generator; and generating, by a signal generation module, electrical signals for neurological stimulation based on the plurality of therapy parameters. The generalized population model and/or the individualized model includes one or more input variables, one or more outputs, and a fitness function configured to evaluate if the one or more outputs satisfies one or more termination criteria. The one or more outputs include at least one of a pain score, a quality-of-life index, and combinations thereof; and/or the one or more termination criteria include at least one of a pain relief improvement, a quality-of-life index improvement, and combinations thereof.

Preferably, the algorithm is configured to evaluate therapy success and to optimize the plurality of therapy parameters until therapy success is reached.

According to an embodiment, therapy success is evaluated according to metrics as for instance a pain score, a quality-of-life index, patient feedback, a sleep score, patient activity levels, or a sensed physiological signal (e.g. heart rate variability or blood pressure).

Accordingly, therapy parameters, such as spinal cord stimulation parameters, are automatically optimized over time by using two different models, namely a generalized population model derived from multiple patients and an individualized model for the patient which is treated using the determined plurality of signal parameters. Thereby, an evolutionary algorithm-derived model can be built to optimize therapy for individual patients (e.g., given the current programmed parameters, if the therapy outcome is not satisfactory, mutate parameters in a relevant manner to create a new parameter set and test it with the patient for a given period of time to assess if it is more effective. Repeat until it meets satisfaction criteria). As a result, the patient's overall experience can be improved, and power consumption of the neurological stimulation system can be reduced.

According to an embodiment, optimizing the plurality of therapy parameters comprises a stochastic search for a therapy parameter by use of the generalized model.

Preferably, the one or more therapy parameters are selected from the group including (or consisting of), an amplitude, a pulse width, a frequency, a duty cycle, a parameter variation range, a program cycle, and an electrode selection.

According to an embodiment, a program cycle is understood as a period of time where a set of different therapy programs is cycled through.

Preferably, the electrical signals are applied to a plurality of implantable electrodes. The term "electrode selection" may refer to a subset of electrodes of the plurality of implantable electrodes which are used at a particular time and/or therapy cycle. **In** other words, an electrode selection includes the electrodes to which the electrical signals are applied.

Preferably, the algorithm, in particular the generalized population model and the individualized model, are based on artificial intelligence. **In** particular, the generalized population model will keep improving with the addition of new patients, e.g., to suggest the best initial therapy parameters for a patient based on the entire pool of first patient data. Likewise, the individualized model will continuously optimize a given patient's parameters over time. The two model levels can interact to improve the outcome, i.e., optimize the therapy parameters).

The term "artificial intelligence" as used throughout the present application may be understood in the sense of software components or software instances which are designed to correctly interpret data, to learn from such data, and to use those learnings to provide a therapy function through flexible adaptation.

According to some embodiments, which can be combined with other embodiments described herein, the method uses machine learning. For example, a neural network can be implemented to determine the plurality of therapy parameters.

The term "machine learning algorithm" as used throughout the present application refers to an algorithm that builds a model based on training data, in order to make predictions or decisions without being explicitly programmed to do so.

A neural network is a based on a collection of connected nodes. A node that receives a signal processes the signal and can signal neurons connected to it. Typically, nodes are aggregated into layers. Different layers may perform different transformations on their inputs. Signals travel from an input layer to an output layer. Such a neural network may be trained by processing examples, each of which contains a known input and result, forming probability-weighted associations between the two, which are stored within the data structure of the neural network. Thus, the neural network learns to perform tasks by considering given examples.

According to some embodiments, which can be combined with other embodiments described herein, the generalized population model and/or the individualized model includes one or more input variables; one or more outputs; and a fitness function configured to evaluate if the one or more outputs satisfies one or more termination criteria. The one or more termination criteria may also be referred to as success or therapy success criteria encompassing one or more objectives with each objective ranked differently depending on collective (i.e. population) or individual preferences or needs
Preferably, the plurality of therapy parameters is determined only if the one or more outputs do not satisfy the one or more termination criteria. In other words, the optimization is continued as long as therapy success has not been reached.

Preferably, the one or more input variables are based on, or correspond to, the one or more therapy parameters. For example, the one or more input variables may include one or more of a stimulation amplitude, a stimulation frequency, a stimulus pulse width, a duty cycling ratio, an electrode selection, a burst envelop frequency, and/or a burst duty cycle ratio.

Preferably, the one or more outputs include at least one of a pain score, a quality-of-life index, patient feedback, and combinations thereof.

Preferably, the pain score is provided as feedback from the patient e.g. via an input device such as a patient remote. The pain score may be defined on a numerical rating scale (NRS) scale from 0-10.

Preferably, the quality-of-life index is derived from one or more metrics collected without direct input from the patient. For example, accelerometry data and derived activity/sleep metrics, physiological measurements (e.g., heart rate, heart rate variability, blood pressure, etc.), user-directed therapy changes, and/or battery usage can be used to determine the quality-of-life index.

The patient feedback (other than the pain score) may be prompted by the patient remote and may include for instance a therapy satisfaction score and/or well-being score.

Preferably, the one or more termination criteria include at least one of a pain relief improvement, a quality-of-life index improvement, a patient feedback improvement, and combinations thereof.

According to some embodiments, which can be combined with other embodiments described herein, the generalized population model and/or the individualized model are based on an evolutionary algorithm, in particular a Differential Evolution algorithm. For example, the generalized population model and/or the individualized model can be a version of a Differential Evolution (DE) algorithm including but not limited to hybrid and population-based DE adapted for this therapy optimization problem.

According to some embodiments, which can be combined with other embodiments described herein, the generalized population model is configured to provide one or more initial therapy parameters to the individualized model and/or guide therapy parameter optimization of the individualized model. In particular, the generalized population model can be trained with the pool of all available patient data, in order to suggest a set of initial parameters for a new patient and/or guide the personalized therapy parameter optimization process with the individualized model e.g. by constraining the parameter space to prevent selection of an unsafe or undesirable combination of therapy parameters and/or providing input.

According to some embodiments, which can be combined with other embodiments described herein, the method further includes determining, by the individualized model, the plurality of (optimized) therapy parameters by mutating a plurality of reference therapy parameters. For example, each parameter may mutate as defined by its probability function constrained by patient safety-related strict rules.

Preferably, the plurality of reference therapy parameters is the plurality of current therapy parameters or are provided by the generalized population model.

According to some embodiments, which can be combined with other embodiments described herein, determining (or optimizing), by the therapy parameter determination module, the plurality of therapy parameters is done at discrete times or continuously. In particular, therapy success can be evaluated on a regular basis, e.g. when the patient is prompted to assess their pain level by their patient remote. Optimization in this case is a process defined in time, with a start and an end, recurring over time. The process can be called a therapy optimization session. A therapy optimization session can be automatically triggered depending on the therapy success evaluation result. Alternatively, therapy success according can be automatically and/or continuously evaluated. Optimization is then an ongoing process at all times, with no discrete boundaries in time like a therapy optimization session would have. This can be referred to as continuous optimization.

According to some embodiments, which can be combined with other embodiments described herein, the neurological stimulation is spinal cord stimulation (SCS).

According to another independent aspect of the present disclosure, a machine-readable storage medium is provided. The machine-readable storage medium includes instructions executable by one or more processors to implement the method for neurological stimulation of the embodiments of the present disclosure.

The machine-readable storage medium may include, for example, semiconductor memory devices such as Electrically Programmable Read-Only Memory (EPROM), Electrically Erasable Programmable Read-Only Memory (EEPROM), and the like. The machine-readable storage medium may be used to tangibly retain computer program instructions or code organized into one or more modules and written in any desired computer programming language. When executed by, for example, one or more processors such computer program code may implement the method for neurological stimulation of the embodiments of the present disclosure.

According to another independent aspect of the present disclosure, a neurological stimulation system is provided. The neurological stimulation system includes a signal generation module connectable to a plurality of implantable electrodes and configured to provide electrical signals to the plurality of implantable electrodes for neurological stimulation, wherein the signal generation module is configured to generate the electrical signals according to a plurality of therapy parameters; and a therapy parameter determination module configured to determine the plurality of therapy parameters using an algorithm. The algorithm includes a generalized population model based on first patient data of multiple patients; and an individualized model based on second patient data of the patient who uses the signal generator.

According to some embodiments, which can be combined with other embodiments described herein, the therapy parameter determination module is included in an implantable signal generator or an external entity connectable to the implantable signal generator.

Preferably, the external entity is selected from the group consisting of a server, a service center, and a cloud.

Preferably, the implantable signal generator and the external entity are connected wirelessly, e.g. via a mobile network, WIFI, NFC, and/or Bluetooth.

According to another independent aspect of the present disclosure, a signal generator for a neurological stimulation system. The signal generator includes a signal generation module connectable to a plurality of implantable electrodes and configured to provide electrical signals to the plurality of implantable electrodes for neurological stimulation, wherein the signal generation module is configured to generate the electrical signals according to a plurality of therapy parameters; and a controller configured to operate the signal generation module, wherein the controller is configured to determine the plurality of therapy parameters using an algorithm. The algorithm includes a generalized population model based on first patient data of multiple patients; and an individualized model based on second patient data of the patient who uses the signal generator.

According to another independent aspect of the present disclosure, a neurological stimulation system is provided. The neurological stimulation system includes a plurality of implantable electrodes configured to deliver electrical signals to neurological tissue, such as a spinal cord; and the signal generator of the embodiments of the present disclosure.

According to some embodiments, which can be combined with other embodiments described herein, the neurological stimulation system is configured for spinal cord stimulation. However, the present disclosure is not limited thereto, and the neurological stimulation system may also be used in other stimulation therapies which use electrical signals that are delivered to electrodes implanted in a patient's body.

Embodiments are also directed at systems/devices for carrying out the disclosed methods and include system/device aspects for performing each described method aspect. These method aspects may be performed by way of hardware components, a computer programmed by appropriate software, by any combination of the two or in any other manner. Furthermore, embodiments disclosed herein are also directed at methods for operating the described device/system. It includes method aspects for carrying out every function of the device/system.

So that the manner in which the above recited features of the present disclosure can be understood in detail, a more particular description of the disclosure, briefly summarized above, may be had by reference to embodiments. The accompanying drawings relate to embodiments of the disclosure and are described in the following:
- FIG. 1: shows a schematic view of a neurological stimulation system according to embodiments of the present disclosure;
- FIG. 2: shows an overview of an algorithm for optimization of therapy parameters according to an embodiment of the present disclosure; and
- FIG. 3: shows a flowchart of an optimization of therapy parameters according to further embodiments of the present disclosure.

Reference will now be made in detail to the various embodiments of the disclosure, one or more examples of which are illustrated in the figures. Within the following description of the drawings, the same reference numbers refer to same components. Generally, only the differences with respect to individual embodiments are described. Each example is provided by way of explanation of the disclosure and is not meant as a limitation of the disclosure. Further, features illustrated or described as part of one embodiment can be used on or in conjunction with other embodiments to yield yet a further embodiment. It is intended that the description includes such modifications and variations.

There is a need to reliably find an optimal set of parameters that achieve satisfying pain relief for each patient, and find the most optimal set of parameters that achieves the best pain relief a patient can experience with SCS, and preferably a set of parameters that also minimizes power consumption to promote the longevity of the stimulator battery as well as improve patient's overall experience by reducing charging frequency in case a rechargeable stimulator is used.

The present disclosure offers patient-specific optimization over time without manual reprogramming. The embodiments of the present disclosure may consider both each patient's past experience, and the pool of past patients' successes to drive optimization and find effective parameters in an effective way. The automatic nature of this approach removes the time-consuming burden from both the medical staff and system clinicians to manually re-program ineffective therapies and addresses the known phenomenon of therapy habituation that can happen over time.

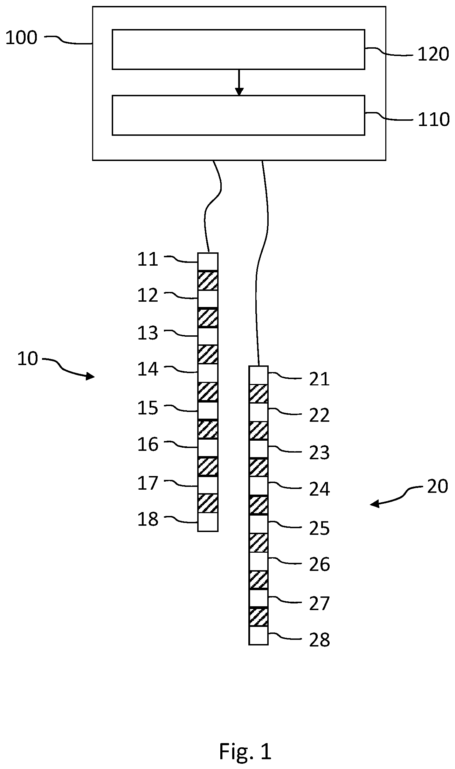

FIG. 1 shows a schematic view of a neurological stimulation system according to embodiments of the present disclosure.

The neurological stimulation system includes a signal generator 100 and a plurality of implantable electrodes configured to deliver electrical signals to neurological tissue. In the example of FIG. 1, the neurological stimulation system is configured for spinal cord stimulation.

In some embodiments, the plurality of implantable electrodes, such as ring electrodes, can be arranged sequentially along one or more elongated leads. In the example of FIG. 1, a plurality of first electrodes 11-18 is arranged sequentially along a first lead 10, and a plurality of second electrodes 21-28 is arranged sequentially along a second lead 20.

The plurality of first electrodes 11-18 and/or the plurality of second electrodes 21-28 are configured for delivering therapy to the patient after implantation. In particular, the plurality of implantable electrodes, such as the plurality of first electrodes 11-18 and/or the plurality of second electrodes 21-28, can be implanted at or near a spinal cord to direct electrical signals into the patient's tissue for spinal cord stimulation.

The signal generator 100 can be implanted subcutaneously and electrically connected to the plurality of implantable electrodes. The signal generator 100 generates electrical signals to be delivered to the patient via the plurality of implantable electrodes. In particular, the signal generator 100 can be a pulse generator. The pulse generator generates the electrical signals having a particular amplitude, pulse width, frequency and/or duty cycle suitable for stimulation therapy.

The signal generator 100 includes a signal generation module 110 connectable to the plurality of implantable electrodes and configured to provide electrical signals to the plurality of implantable electrodes for neurological stimulation, wherein the signal generation module 110 is configured to generate the electrical signals according to a plurality of therapy parameters; and a controller 120 configured to operate the signal generation module 110. The controller 120 is configured to determine the plurality of therapy parameters using an algorithm which includes a generalized population model based on first patient data of multiple patients; and an individualized model based on second patient data of the patient who uses the signal generator 100.

The algorithm can make multiple iterations of therapy programming and integrate the result in the two levels of the models (general and personalized), in order to ultimately find the optimal set of therapy parameters.

In the example of FIG. 1, the controller 120 determines or optimizes the plurality of therapy parameters. However, the present disclosure is not limited thereto, and the algorithm can be stored and operate on a remote digital location (e.g. server, cloud, service center), collecting system data remotely (e.g. via a service center or cloud).

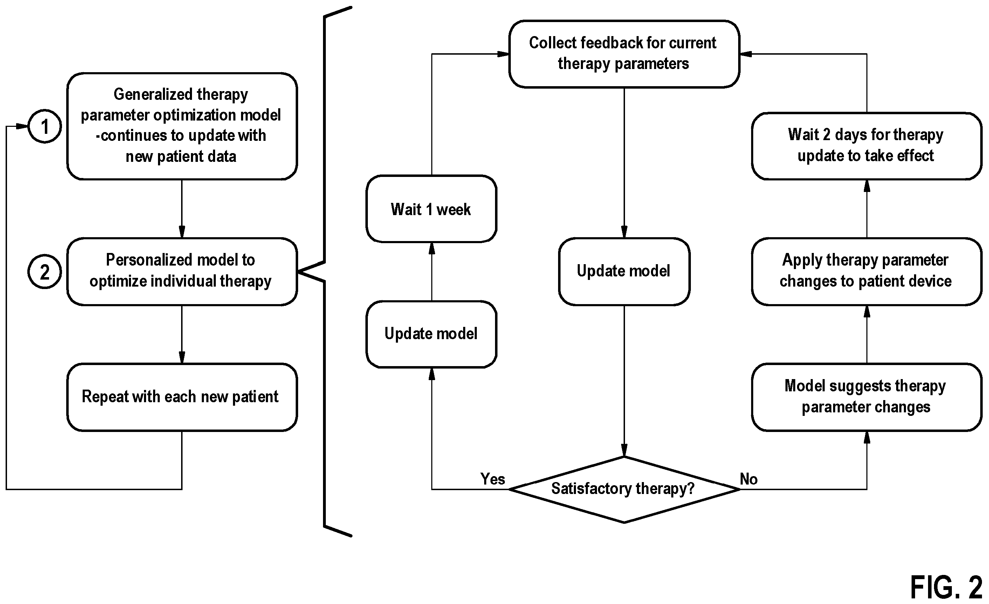

FIG. 2 shows an overview of an algorithm for optimization of therapy parameters according to an embodiment of the present disclosure.

In the following, the model structure, the generalized population model, and the individualized model are described.

### 1. Model structure (generalized population model and individualized model)

The model may include a set of defined input variables X (features) and one or more outputs Y (labels).

The set of input variables X may include one or more of a stimulation amplitude, a stimulation frequency, a stimulus pulse width, a duty cycling ratio (time when stimulation is on vs. off), an electrode configuration (or electrode selection), a burst envelop frequency (burst interval), a program cycle (program changes occurring at regular time intervals), and/or a burst duty cycle ratio (burst on vs. off).

The one or more outputs Y can be defined in multiple different ways, some examples of which are described in the following.

In a first embodiment, the output is a pain score (e.g., NRS, scale 0-10) provided by the patient when prompted by a patient remote.

In a second embodiment, the output is a quality-of-life index. The quality-of-life index may be derived from one or more of the following metrics collected by the neurological stimulation system, none of which require the patient's direct input:
- accelerometry data and derived activity/sleep metrics such as step count, activity counts, and/or sleep quality/duration; and/or
- physiological measurements such as heart rate, heart rate variability, and/or blood pressure; and/or
- user-directed therapy changes reflecting the need for the patient to adjust therapy; and/or
- battery usage (frequent recharging times impacts the patient's overall treatment experience).

In a third embodiment, the output is a combination of the outputs of the first and second embodiments, with at least one output from each (e.g., a combination of patient remote-prompted NRS, step count, sleep quality and heart rate variability).

In a fourth embodiment, the output is patient feedback other than pain score, prompted by the patient remote, such as a 'therapy satisfaction score', 'well-being score' or 'sleep-quality score'.

In addition to the input variables and the output(s), the model may further include a target function f such as Y = f(X), where f is not explicitly known, since it represents the real world's SCS action. There is at least one defined feature and one defined output.

In addition to the input variables and the output(s) and optionally the target function, the model may further include a fitness function to evaluate if the estimated model parameters (updated therapy parameters) satisfy optimization success (termination) criteria. Exemplary optimization success criteria can be defined as follows.

In the first embodiment, the optimization success (termination) criteria may include a pain relief improvement (success = pain relief improvement), e.g.:
- satisfying pain relief: e.g. at least 50% between the NRS from 2 to 7 days after therapy parameter update, minus the baseline pre-treatment NRS; and/or
- improved pain relief: e.g. at least 20% between pre- and post-therapy parameter update.

It is noted that "satisfying" and "improved" pain relief are two criteria that can be used separately or in combination to optimize the therapy. Satisfying pain relief shall be used to indicate to the model when the minimum target of satisfying pain relief has been reached, and improved pain relief shall be used for further therapy improvement after satisfying pain relief criterion has been reached.

In the second embodiment, the optimization success (termination) criteria may include an improvement corresponding to at least a quality-of-life index (success = quality-of-life index), such as a quality-of-life index improvement of at least 20-30% for satisfying outcome and 10-20% for improved outcome.

In the third embodiment, the optimization success (termination) criteria may include a combined (e.g., weighted average) quality-of-life index and pain score improvement, such as a quality-of-life index and pain score improvement of at least 30% for satisfying outcome and 20% for improved outcome.

In the fourth embodiment, the optimization success (termination) criteria may include a patient feedback improvement (success = patient feedback improvement), such as a patient feedback improvement of at least 20-30% for satisfying outcome and 10-20% for improved outcome.

### 2. Generalized therapy parameter optimization model

In a preferred embodiment, the generalized population model can be trained with the pool of all available patient data, in order to suggest a set of initial parameters for a new patient and/or guide the personalized therapy parameter optimization process (with the patient-specific personalized model), by (1) constraining the parameter space to prevent selection of an unsafe or undesirable combination of therapy parameters and (2) providing input.

More specifically, according to an embodiment, the method of training the generalized population model further comprises the steps of: (3) preventing overfitting effects (i.e. finding optimal parameters that are specific to a small subset of patient preferences and incorrectly generalizing them to apply to all patients), which reduces the variance of the generalized (population) parameter estimates", (4) providing optimal initial conditions for further individualized optimization process.

In another embodiment, that initial parameters for a given patient can be set by an expert, e.g. clinician or physician, and do not require a generalized therapy parameter optimization model.

An exemplary model type that suits this therapy parameter optimization is an evolutionary algorithm, in which multiple model options are available. In all embodiments, the preferred model can be a version of a Differential Evolution (DE) algorithm including but not limited to hybrid and population-based DE adapted for this therapy optimization problem. The algorithm operation to optimize the therapy parameters of a new patient is described in the next paragraph. In other embodiments, a genetic algorithm model or particle swarm optimization can be used.

### 3. Patient-specific personalized therapy parameter optimization model

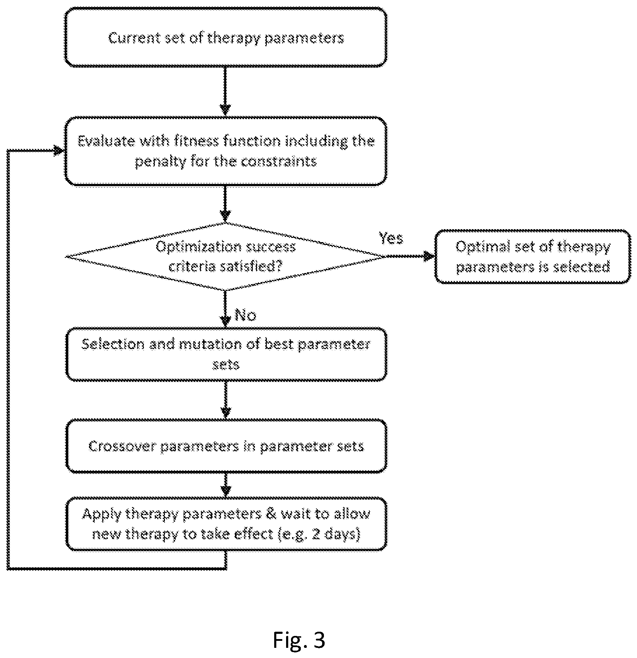

An exemplary DE algorithm-derived optimization, including but not limited to a self-adaptive and gradient-based hybrid DE method, to improve therapy outcome for a given patient is illustrated FIG. 3.

The optimization algorithm can either be continuous, starting after the patient is implanted with a stimulation system and their system is initially programmed or after a given point in time, with no scheduled ending, or it can be executed in discrete sessions happening at regular or irregular intervals over time.

### Optimization process description (illustrated in FIG. 3)

The optimization process starts, and the algorithm evaluates the current set of parameters of a patient's device using the fitness function (e.g. set by the generalized model or by a clinician/expert). If the fitness function output meets the optimization success criteria, the set of parameters is left unchanged, and the model is updated to reflect this instance.

If the fitness function output does not meet the optimization success criteria, a new set of parameters can be created using the following steps:
a. The best parameter set (according to the fitness function) is selected based on the generalized model in one embodiment, or among the past attempts in another embodiment. If this is the first attempt, then it is the current set of parameters.
b. The selected parameter set undergoes one or more mutations: each parameter may mutate as defined by its probability function constrained by patient safety-related strict rules. Model hyperparameter (also called DE control parameters) are adjusted according to self-adaptive DE to account for individual differences in SCS responses. Safety-related strict rules include preventing overstimulation and/or unsafe charge delivery by forbidding a parameter space where there is one or a combination of the following:
   - electrical charge is too high,
   - amplitude is too high, and/or
   - the amplitude/pulse width pair exceeds the strength-duration curve safety margin, which may be based on measured the patient's perception threshold:
c. The selected parameter sets that may contain mutations undergo crossover to create new combinations.

Thereafter the patient's therapy parameters are updated with the new offspring set. The algorithm may then wait between 1 day and 1 week (e.g. 2 days) before re-evaluating the therapy outcome using the fitness function.

The optimization process then starts over again by again evaluating the current set of parameters of a patient's device using the fitness function.

### Optimization process definitions

In item 1 above, first to fourth embodiments for the definition of optimization success have been described.

In a fifth embodiment, which can include any of the elements of previous first to fourth embodiments, therapy success according to the fitness function can be evaluated on a regularly basis, e.g. when the patient is prompted to assess their pain level by their patient remote. Optimization in this case is a process defined in time, with a start and an end, recurring over time. The process can be called a therapy optimization session. A therapy optimization session can be automatically triggered depending on the therapy success evaluation result.

In a sixth embodiment, which can include any of the elements of previous first to fourth embodiments, therapy success according to the fitness function can be automatically and/or continuously evaluated using the fitness function. Optimization is then an ongoing process at all times, with no discrete boundaries in time like a therapy optimization session would have. This can be referred to as continuous optimization. In this case, mutations can be of the adaptive type to reduce mutation probabilities in parameters sets that score already high with the fitness function.

### Optimization success definition

In one embodiment, the goal of the therapy optimization process (optimization session or continuous optimization) is to search and optimize parameters until satisfying therapy success is reached, maintained or returned to (e.g. after pain relief has started fading).

In another embodiment, the goal of the therapy optimization process (optimization session or continuous optimization) is to continuously optimize parameters of an already satisfying pain relief to further increase, maintain or return to an improved therapy success (e.g. after pain relief has started fading but is still meeting the satisfying therapy success level).

It is noted that therapy success can include both pain relief and patient experience, which is improved by non-pain-dependent factors such as decreased charging frequencies.

### Optimization execution rules

In one embodiment, therapy optimization session asks for patient authorization before executing, e.g. via their patient remote.

In another embodiment, therapy optimization session starts without patient authorization, and may or may not notify them of its execution.

### Optimization algorithm implementation

The algorithm can be implemented either on the system or be stored and operate on a remote digital location (e.g. server, cloud, service center), collecting system data remotely (e.g. via a service center or cloud).

### Multi-sequence alignment optimization

The embodiments of the present disclosure entail a genetic optimization which tests one set of amplitude, pulse width, stimulation frequency, electrode selection, and cycling parameters, and evaluates changes in pain score or objective behavioral or physiological metrics to evaluate a fitness optimization function to determine preferential genetic combinations. It is recognized that during this optimization search, certain parameter sets are possible which provide little or no therapeutic effect, and as such may allow the patients pain to return during the evaluation period. In the case of such an event, the system may provide the patient an option to declare a fail for the current parameter combination prior to the automatic time-out of an evaluation period, and advance to the next tested set.

In addition, the fitness function should take into account power delivery of stimulation parameter sets as a negative weighting factor, such that between two stimulation parameter sets which provide the same pain relief and/or quantifiable input metrics, the lower power requiring stimulation parameter set is preferred by the fitness function.

Furthermore, testing of a single parameter set at a time over the course of a few days or a week will require dozens of weeks or a year to iterate the DE algorithm to search for more optimal solution if selection pressure is low that favors random global search over local search. Instead, a preferred embodiment is to deliver stimulation of combinations of parameters for much shorter discrete time periods in a multi-sequence series manner, and to allow the DE algorithm to utilize existence of a parameter combination in the sequence, and specific location of a parameter set near a local optimum in the sequence to be further parameters for the local optimization of the genetic code.

For example, parameter sets A - T are 20 possible sets for initial testing. A subset S1 of 6 of these parameter sets is selected and delivered over the course of an hour whereby each parameter set is delivered within that hour for 10 minutes, and the sequence repeats. Following several days of testing and feedback, subset S1 is mutated to S2, containing a combination of a portion of the S1 subset and a portion of the remaining untested set of 20 parameter sets. Every evolutionary cycle therefore allows testing of performance of multiple parameter sets, accepting that some parameter sets will provide similar, better, or worse performance than others. The group of parameters is thus iterated until the preferred set of 6 parameter sets out of the available 20 is then determined. From this preferred group, further mutations may be created over time to allow a continuous year-over-year automatic global optimization process which ensures that a substantial portion of the time therapy is being delivered with a preferred, successful parameter set, and a portion of the time that therapy is being delivered, it is delivered with a test mutated parameter set.

This solution of multi-sequence alignment optimization utilizes the physiological pain relief carryover effect, in which pain relief is maintained over the course of hours even after sub-optimal stimulation is delivered. Delivering preferred stimulation periods intermixed with periods of unknown test stimulation allows for less abrupt changes in pain relief and sensation as various parameter combinations are tested.

While the foregoing is directed to embodiments of the disclosure, the scope of the invention is determined by the claims that follow.

## Claims

1. Neurological stimulation system, comprising:
a signal generation module (110) connectable to a plurality of implantable electrodes (11-18, 21-28) and configured to provide electrical signals to the plurality of implantable electrodes (11-18, 21-28) for neurological stimulation, wherein the signal generation module (110) is configured to generate the electrical signals according to a plurality of therapy parameters; and
a therapy parameter determination module configured to determine the plurality of therapy parameters using an algorithm which includes:
- determining, by the therapy parameter determination module, a plurality of therapy parameters using an algorithm which includes a generalized population model based on first patient data of multiple patients and an individualized model based on second patient data of the patient who uses the signal generator (100); and
- generating, by the signal generation module (110), electrical signals for neurological stimulation based on the plurality of therapy parameters, wherein the generalized population model and/or the individualized model includes:
• one or more input variables;
• one or more outputs; and
**characterized in that** the the generalized population model and/or the individualized model further includes:
• a fitness function configured to evaluate if the one or more outputs satisfies one or more termination criteria,
and wherein:
• the one or more outputs include at least one of a pain score, a quality-of-life index, and combinations thereof; and/or
• the one or more termination criteria include at least one of a pain relief improvement, a quality-of-life index improvement, and combinations thereof, and wherein the generalized population model and/or the individualized
model are based on a Differential Evolution algorithm.

2. The system of claim 1, wherein the algorithm further includes to evaluate therapy success and to optimize the plurality of therapy parameters until therapy success is reached.

3. The system of claim 1 or 2, wherein the algorithm further includes to select the one or more therapy parameters from the group consisting of an amplitude, a pulse width, a frequency, a duty cycle, a parameter variation range, a program cycle, and an electrode selection.

4. The system of one of the preceding claims, wherein the algorithm further includes to determine the plurality of therapy parameters only if the one or more outputs do not satisfy the one or more termination criteria.

5. The system of any one of the preceding claims, wherein the algorithm further includes that the generalized population model is configured to provide one or more initial therapy parameters to the individualized model and/or guide therapy parameter optimization of the individualized model.

6. The system of anyone of the preceding claims, wherein the algorithm further includes determining, by the individualized model, the plurality of therapy parameters by mutating a plurality of reference therapy parameters.

7. The system of claim 6, wherein the algorithm further includes that the plurality of reference therapy parameters is the plurality of current therapy parameters or are provided by the generalized population model.

8. The system of anyone of the preceding claims, wherein the therapy parameter determination module is configured to determine if the plurality of therapy parameters is done at discrete times or continuously.

9. The system of anyone of the preceding claims, wherein the neurological stimulation is spinal cord stimulation.

10. The system of anyone of the preceding claims, wherein the therapy parameter determination module is included in an implantable signal generator or an external entity connectable to the implantable signal generator.

11. A machine-readable storage medium, comprising instructions executable by one or more processors to implement a method for neurological stimulation comprising the steps of:
- determining, by a therapy parameter determination module, a plurality of therapy parameters using an algorithm which includes a generalized population model based on first patient data of multiple patients and an individualized model based on second patient data of the patient who uses the signal generator; and
- generating, by a signal generation module, electrical signals for neurological stimulation based on the plurality of therapy parameters,
**characterized in that**
the generalized population model and/or the individualized model includes:
• one or more input variables;
• one or more outputs; and
• a fitness function configured to evaluate if the one or more outputs satisfies one or more termination criteria,
and wherein:
• the one or more outputs include at least one of a pain score, a quality-of-life index, and combinations thereof; and/or
• the one or more termination criteria include at least one of a pain relief improvement, a quality-of-life index improvement, and combinations thereof, and wherein the generalized population model and/or the individualized
model are based on a Differential Evolution algorithm.

## Patentansprüche

1. Neurologisches Stimulationssystem, umfassend:
ein Signalgenerierungsmodul (110), das mit einer Vielzahl von implantierbaren Elektroden (11-18, 21-28) verbindbar und so konfiguriert ist, dass es elektrische Signale an die Vielzahl von implantierbaren Elektroden (11-18, 21-28) zur neurologischen Stimulation liefert, wobei das Signalgenerierungsmodul (110) so konfiguriert ist, dass es die elektrischen Signale gemäß einer Vielzahl von Therapieparametern erzeugt; und
einem Modul zur Bestimmung von Therapieparametern, das so konfiguriert ist, dass es die Vielzahl von Therapieparametern unter Verwendung eines Algorithmus bestimmt, der Folgendes umfasst:
- das Bestimmen einer Vielzahl von Therapieparametern durch das Modul zur Bestimmung von Therapieparametern unter Verwendung eines Algorithmus, der ein verallgemeinertes Populationsmodell auf der Grundlage erster Patientendaten mehrerer Patienten und ein individualisiertes Modell auf der Grundlage zweiter Patientendaten des Patienten, der den Signalgenerator verwendet, umfasst; und
- Erzeugen von elektrischen Signalen für die neurologische Stimulation durch das Signalerzeugungsmodul auf der Grundlage der Vielzahl von Therapieparametern,
wobei
das verallgemeinerte Populationsmodell und/oder das individualisierte Modell umfasst:
• eine oder mehrere Eingangsvariablen;
• eine oder mehrere Ausgabegrößen; und
**dadurch gekennzeichnet, dass** das verallgemeinerte Populationsmodell und/oder das individualisierte Modell ferner umfasst:
• eine Fitnessfunktion, die so konfiguriert ist, dass sie bewertet, ob die eine oder mehrere Ausgänge ein oder mehrere Beendigungskriterien erfüllen,
und wobei:
• die eine oder mehrere Ausgabegrößen mindestens einen Schmerzscore, einen Lebensqualitätsindex, und Kombinationen davon umfassen; und/oder
• die ein oder mehrere Beendigungskriterien mindestens eines der folgenden Elemente umfassen: eine Verbesserung der Schmerzlinderung, eine Verbesserung des Lebensqualitätsindexes, und Kombinationen davon,
und wobei das verallgemeinerte Populationsmodell und/oder das individualisierte Modell auf einem Differential-Evolution-Algorithmus basieren.

2. Das System nach Anspruch 1, wobei der Algorithmus ferner die Bewertung des Therapieerfolgs und die Optimierung der Vielzahl von Therapieparametern umfasst, bis der Therapieerfolg erreicht ist.

3. Das System nach Anspruch 1 oder 2, wobei der Algorithmus ferner die Auswahl des einen oder der mehreren Therapieparameter aus der Gruppe umfasst, die aus einer Amplitude, einer Pulsbreite, einer Frequenz, einem Tastverhältnis, einem Parametervariationsbereich, einem Programmzyklus und einer Elektrodenauswahl besteht.

4. Das System nach einem der vorstehenden Ansprüche, wobei der Algorithmus ferner umfasst, die Vielzahl von Therapieparametern nur dann zu bestimmen, wenn die eine oder mehrere Ausgabegrößen die ein oder mehreren Beendigungskriterien nicht erfüllen.

5. Das System nach einem der vorstehenden Ansprüche, wobei der Algorithmus ferner vorsieht, dass das verallgemeinerte Populationsmodell so konfiguriert ist, dass es dem individualisierten Modell einen oder mehrere anfängliche Therapieparameter bereitstellt und/oder die Optimierung der Therapieparameter des individualisierten Modells steuert.

6. Das System nach einem der vorstehenden Ansprüche, wobei der Algorithmus ferner umfasst, dass das individualisierte Modell die Vielzahl von Therapieparametern durch Mutation einer Vielzahl von Referenztherapieparametern bestimmt.

7. System nach Anspruch 6, wobei der Algorithmus ferner vorsieht, dass die Vielzahl von Referenztherapieparametern die Vielzahl von aktuellen Therapieparametern ist oder durch das verallgemeinerte Populationsmodell bereitgestellt wird.

8. Das System nach einem der vorstehenden Ansprüche, wobei das Modul zur Bestimmung der Therapieparameter so konfiguriert ist, dass es bestimmt, ob die Vielzahl von Therapieparametern zu diskreten Zeitpunkten oder kontinuierlich ermittelt wird.

9. Das System nach einem der vorstehenden Ansprüche, wobei die neurologische Stimulation eine Rückenmarksstimulation ist.

10. Das System nach einem der vorstehenden Ansprüche, wobei das Modul zur Bestimmung der Therapieparameter in einem implantierbaren Signalgenerator oder einer externen Einheit enthalten ist, die mit dem implantierbaren Signalgenerator verbunden werden kann.

11. Ein maschinenlesbares Speichermedium, das Anweisungen umfasst, die von einem oder mehreren Prozessoren ausgeführt werden können, um ein Verfahren zur neurologischen Stimulation zu implementieren, das die folgenden Schritte umfasst:
- Bestimmen einer Vielzahl von Therapieparametern durch ein Modul zur Bestimmung von Therapieparametern unter Verwendung eines Algorithmus, der ein verallgemeinertes Populationsmodell auf der Grundlage erster Patientendaten mehrerer Patienten und ein individualisiertes Modell auf der Grundlage zweiter Patientendaten des Patienten, der den Signalgenerator verwendet, umfasst; und
- Erzeugen elektrischer Signale zur neurologischen Stimulation durch ein Signalgenerierungsmodul auf der Grundlage der Vielzahl von Therapieparametern,
**dadurch gekennzeichnet, dass**
das verallgemeinerte Populationsmodell und/oder das individualisierte Modell umfasst:
• eine oder mehrere Eingangsvariablen;
• eine oder mehrere Ausgabegrößen; und
• eine Fitnessfunktion, die so konfiguriert ist, dass sie bewertet, ob die eine oder mehrere Ausgänge ein oder mehrere Beendigungskriterien erfüllen,
und wobei:
• die eine oder mehrere Ausgabegrößen mindestens einen Schmerzscore, einen Lebensqualitätsindex, und Kombinationen davon umfassen; und/oder
• die ein oder mehrere Beendigungskriterien mindestens eines der folgenden Elemente umfassen: eine Verbesserung der Schmerzlinderung, eine Verbesserung des Lebensqualitätsindexes, und Kombinationen davon,
und wobei das verallgemeinerte Populationsmodell und/oder das individualisierte Modell auf einem Differential-Evolution-Algorithmus basieren.

## Revendications

1. Système de stimulation neurologique, comprenant :
un module de génération de signaux (110) pouvant être connecté à une pluralité d'électrodes implantables (11-18, 21-28) et configuré pour fournir des signaux électriques à ladite pluralité d'électrodes implantables (11-18, 21-28) en vue d'une stimulation neurologique, dans lequel le module de génération de signaux (110) est configuré pour générer les signaux électriques en fonction d'une pluralité de paramètres thérapeutiques ; et
un module de détermination des paramètres thérapeutiques configuré pour déterminer la pluralité de paramètres thérapeutiques à l'aide d'un algorithme qui comprend :
- la détermination, par le module de détermination des paramètres thérapeutiques, d'une pluralité de paramètres thérapeutiques à l'aide d'un algorithme qui comprend un modèle de population généralisé basé sur des premières données de patients provenant de plusieurs patients et un modèle individualisé basé sur des secondes données de patient provenant du patient qui utilise le générateur de signaux ; et
- la génération, par le module de génération de signaux, de signaux électriques pour la stimulation neurologique sur la base de la pluralité de paramètres thérapeutiques,
dans lequel
le modèle de population généralisé et/ou le modèle individualisé comprend :
• une ou plusieurs variables d'entrée ;
• une ou plusieurs sorties ; et **caractérisé en ce que** le modèle de population généralisé et/ou le modèle individualisé comprend en outre :
• une fonction d'aptitude configurée pour évaluer si la ou les sorties satisfont à un ou plusieurs critères de terminaison,
et dans lequel :
• la ou les sorties comprennent au moins l'un parmi un score de douleur, un indice de qualité de vie, et des combinaisons de ceux-ci ; et/ou
• le ou les critères de terminaison comprennent au moins l'un parmi une amélioration du soulagement de la douleur, une amélioration de l'indice de qualité de vie, et des combinaisons de ceux-ci,
et dans lequel le modèle de population généralisé et/ou le modèle individualisé sont basés sur un algorithme d'évolution différentielle.

2. Système selon la revendication 1, dans lequel l'algorithme comprend en outre l'évaluation du succès du traitement et l'optimisation de la pluralité de paramètres thérapeutiques jusqu'à ce que le succès du traitement soit atteint.

3. Le système selon la revendication 1 ou 2, dans lequel l'algorithme comprend en outre la sélection d'un ou plusieurs paramètres thérapeutiques parmi le groupe constitué d'une amplitude, d'une largeur d'impulsion, d'une fréquence, d'un rapport cyclique, d'une plage de variation des paramètres, d'un cycle de programme et d'une sélection d'électrodes.

4. Système selon l'une des revendications précédentes, dans lequel l'algorithme comprend en outre la détermination de la pluralité de paramètres thérapeutiques uniquement si la ou les sorties ne satisfont pas au ou aux critères de terminaison.

5. Système selon l'une quelconque des revendications précédentes, dans lequel l'algorithme comprend en outre que le modèle de population généralisé est configuré pour fournir un ou plusieurs paramètres thérapeutiques initiaux au modèle individualisé et/ou pour guider l'optimisation des paramètres thérapeutiques du modèle individualisé.

6. Système selon l'une quelconque des revendications précédentes, dans lequel l'algorithme comprend en outre la détermination, par le modèle individualisé, de la pluralité de paramètres thérapeutiques en modifiant une pluralité de paramètres thérapeutiques de référence.

7. Système selon la revendication 6, dans lequel l'algorithme prévoit en outre que la pluralité de paramètres thérapeutiques de référence correspond à la pluralité de paramètres thérapeutiques actuels ou est fournie par le modèle de population généralisé.

8. Système selon l'une quelconque des revendications précédentes, dans lequel le module de détermination des paramètres thérapeutiques est configuré pour déterminer si la pluralité de paramètres thérapeutiques est déterminée à des moments discrets ou en continu.

9. Système selon l'une quelconque des revendications précédentes, dans lequel la stimulation neurologique est une stimulation de la moelle épinière.

10. Système selon l'une quelconque des revendications précédentes, dans lequel le module de détermination des paramètres thérapeutiques est inclus dans un générateur de signaux implantable ou dans une entité externe pouvant être connectée au générateur de signaux implantable.

11. Support de stockage lisible par machine, comprenant des instructions exécutables par un ou plusieurs processeurs pour mettre en œuvre un procédé de stimulation neurologique comprenant les étapes suivantes :
- déterminer, à l'aide d'un module de détermination des paramètres thérapeutiques, une pluralité de paramètres thérapeutiques à l'aide d'un algorithme qui comprend un modèle de population généralisé basé sur des premières données de patients provenant de plusieurs patients et un modèle individualisé basé sur des deuxièmes données de patients provenant du patient qui utilise le générateur de signaux ; et
- générer, par un module de génération de signaux, des signaux électriques pour la stimulation neurologique sur la base de la pluralité de paramètres thérapeutiques,
**caractérisé en ce que**
le modèle de population généralisé et/ou le modèle individualisé comprend :
• une ou plusieurs variables d'entrée ;
• une ou plusieurs sorties ; et
• une fonction d'aptitude configurée pour évaluer si la ou les sorties satisfont à un ou plusieurs critères de terminaison,
et dans lequel :
• la ou les sorties comprennent au moins l'un parmi un score de douleur, un indice de qualité de vie, et des combinaisons de ceux-ci ; et/ou
• le ou les critères de terminaison comprennent au moins l'un parmi une amélioration du soulagement de la douleur, une amélioration de l'indice de qualité de vie, et des combinaisons de ceux-ci,
et dans lequel le modèle de population généralisé et/ou le modèle individualisé sont basés sur un algorithme d'évolution différentielle.
